# EUROPÄISCHE PATENTSCHRIFT

(11) **EP 4 210 631 B1**
(45) Veröffentlichungstag und Bekanntmachung des Hinweises auf die Patenterteilung: **24.07.2024**
(21) Anmeldenummer: 21769362.1
(22) Anmeldetag: 23.08.2021
(51) Int. Cl.: A61F 2/16

(54) **AKKOMMODATIVE INTRAOKULARLINSE**
ACCOMMODATIVE INTRAOCULAR LENS
LENTILLE INTRAOCULAIRE ADAPTATIVE

(30) Priorität: 09.09.2020 DE 102020123518
(43) Veröffentlichungstag der Anmeldung: 19.07.2023
(73) Patentinhaber: Carl Zeiss Meditec AG, 07745 Jena (DE)
(72) Erfinder: SCHREIBER, Benjamin, 10551 Berlin (DE); WOLFSTEIN, André, 13507 Berlin (DE); WOLF, Uwe, 99441 Magdala (DE); BUCHHEISTER, Jan, 07751 Jena (DE); LUQUE, Sergio, 1220 Wien (AT)
(74) Vertreter: PATERIS Patentanwälte PartmbB
(86) Internationale Anmeldenummer: PCT/EP2021/073251
(87) Internationale Veröffentlichungsnummer: WO 2022/053299

(56) Entgegenhaltungen:
- DE-B3- 102018 212 774

## Beschreibung

Die Erfindung betrifft eine akkommodative Intraokularlinse.

Mit einer natürlichen Augenlinse ist es möglich, in der Ferne und in der Nähe Gegenstände scharf zu sehen. Ermöglicht wird dies dadurch, dass die Augenlinse in ihrer Form und damit die Brechkraft verändert werden kann. Die Augenlinse ist in einem Kapselsack enthalten, der an Zonulafasern aufgehängt ist, welche wiederum mit einem Ziliarmuskel verbunden sind. Wenn der Ziliarmuskel sich entspannt, werden die Zonulafasern gestrafft, wodurch der Kapselsack gestreckt wird. Die Formänderung des Kapselsackes bewirkt bei einer weichen Augenlinse, dass diese sich ebenfalls in ihrer Form verändert. Mit zunehmender Streckung des Kapselsackes wird die Augenlinse zunehmend abgeflacht. Dadurch ändert sich die Brechkraft der Augenlinse. Eine abgeflachte Augenlinse führt zu einer geringeren Brechkraft, sodass eine scharfe Fernsicht ermöglicht wird. Dieser Vorgang ist reversibel, sodass bei einem angespannten Ziliarmuskel die Zonulafasern erschlaffen und der Kapselsack weniger stark gestreckt ist. Damit nimmt die Augenlinse eine Form an, die stärker gewölbt ist, sodass eine höhere Refraktion erreicht wird. Dadurch ist es möglich, Gegenstände in der Nähe scharf zu sehen. Diese Variation in der Schärfenebene wird als Akkommodation bezeichnet.

Es ist normal, dass mit zunehmendem Alter die Augenlinse an Elastizität verliert. Die Augenlinse ist dann weniger in der Lage, ihre Form als Reaktion auf eine Kontraktion des Ziliarmuskels zu ändern. Dies bewirkt, dass ein Fokussieren auf nahe Gegenstände immer schwerer gelingt. Dieser Zustand wird mit Presbyopie bezeichnet. Durch das Tragen einer Brille oder einer Kontaktlinse ist es möglich, die fehlende Brechkraft zu kompensieren. Mit zunehmendem Alter wird die Augenlinse jedoch zunehmend inelastischer bis hart und kann zudem eintrüben. In der Medizin wird eine solcher Zustand der Augenlinse mit Katarakt oder "grauer Star" bezeichnet. Ein Brillenglas kann die Folgen einer Eintrübung der Augenlinse nicht kompensieren, sodass es üblich geworden ist, die eingetrübte Augenlinse chirurgisch zu entfernen. Dazu wird z.B. eine mit Ultraschall schwingende Nadel in das Auge eingeführt und die harte und eingetrübte Augenlinse in kleine Partikel zertrümmert. Dieser Vorgang wird als Phakoemulsifikation bezeichnet. Die Partikel werden nach einer solchen Phakoemulsifikation abgesaugt, bis der Kapselsack von der natürlichen Augenlinse befreit ist. Um wieder ein gutes Sehen zu ermöglichen, wird anschließend eine künstliche Augenlinse in den Kapselsack implantiert. Diese künstliche Augenlinse wird als Intraokularlinse bezeichnet.

Die künstliche Augenlinse ist üblicherweise eine Linse mit einem einzigen Brennpunkt (monofokal), sodass ein Patient nach einer Implantation einer künstlichen Augenlinse eine Brille oder eine Kontaktlinse benötigt, um sowohl in der Ferne als auch in der Nähe scharf zu sehen. Es gibt jedoch auch Überlegungen, die künstliche Augenlinse so zu gestalten, dass eine Akkommodation mit einer sich verändernden Schärfenebene möglich ist. Eine solche künstliche Augenlinse wird auch als akkommodative Intraokularlinse bezeichnet. Das Anspannen oder Entspannen eines Ziliarmuskels soll es ermöglichen, die Form der Intraokularlinse und damit deren Brechkraft zu verändern. Problematisch dabei ist jedoch, dass es schwierig ist, eine Kraft von dem Kapselsack auf die Intraokularlinse zu übertragen, um die Form der Intraokularlinse zu verändern.

DE 10 2018 212 774 B3 betrifft eine akkommodative Intraokularlinse.

Aufgabe der Erfindung ist es daher, eine akkommodative Intraokularlinse zu schaffen, mit der eine Kraft gut von einem Kapselsack auf die Intraokularlinse übertragen werden kann.

Die erfindungsgemäße akkommodative Intraokularlinse zum Einbringen in den Kapselsack eines Auges weist auf:
- einen ersten Linsenteil, welcher aufweist:
- einen für Licht transparenten Optikkörper mit einer optischen Achse, einer distalen Optikkörperoberfläche und einer proximalen Optikkörperoberfläche,
- eine mit dem Optikkörper fest verbundene Haptik,
- eine mit der Haptik und/oder dem Optikkörper fest verbundene flexible Membran, die benachbart zur distalen Optikkörperoberfläche angeordnet ist, zusammen mit der distalen Optikkörperoberfläche einen Hohlraum begrenzt und transparent für das Licht ist, und
- einen zweiten Linsenteil, der einen Hohlzylinder mit einem distalen Ende und einem proximalen Ende aufweist und der sich mit dem ersten Linsenteil lösbar koppeln lässt, wodurch die Intraokularlinse in einen Kopplungszustand bringbar ist, in dem der zweite Linsenteil an einer distalen Seite des ersten Linsenteils angeordnet ist und der Hohlzylinder eingerichtet ist, durch ein Längsverlagern des Hohlzylinders parallel zu der optischen Achse, die Membran zu verformen, wobei der erste Linsenteil eine Mehrzahl von Biegeelementen aufweist, die außerhalb des Hohlraums an der Membran angebracht sind und die in dem Kopplungszustand ein proximales Ende des Hohlzylinders kontaktieren und der Hohlzylinder an seiner Außenseite eine Außenfläche aufweist, die ein in Radialrichtung bezüglich der Achse des Hohlzylinders außenliegendes Ende des Hohlzylinders ist, wobei der Hohlzylinder eine Auflagefläche aufweist, die eingerichtet ist, von dem Kapselsack kontaktiert zu werden, benachbart zu einem proximalen Ende der Außenfläche angeordnet ist und mit der Außenfläche einen Winkel einschließt, der kleiner als 180° ist.

Um die Intraokularlinse in den Kapselsack eines Auges einzusetzen, ist es denkbar, einen möglichst kreisrunden Teil des Kapselsacks herauszuschneiden. Dadurch entsteht eine Schnittkannte, wobei die Schnittkante auch Rhexis genannt wird. Es werden zuerst der erste Linsenteil und anschließend der zweite Linsenteil in den Kapselsack eingesetzt. Bei dem Einsetzen des ersten Linsenteils kommt die Haptik mit dem Kapselsack in Eingriff, wodurch der Optikkörper mittig in dem Kapselsack angeordnet wird. Der zweite Linsenteil wird anschließend so in den Kapselsack angeordnet, dass die Intraokularlinse in den Kopplungszustand gelangt. Anschließend kann das distale Ende des Hohlzylinders in den herausgeschnittenen Teil des Kapselsacks eingebracht werden, wodurch der Kapselsack die Auflagefläche kontaktiert. Dadurch ist eine gute mechanische Kopplung zwischen dem Kapselsack und dem Hohlzylinder geschaffen. Dadurch kann eine Kraft effektiv von dem Kapselsack auf den Hohlzylinder übertragen werden. Anschließend kann die Kraft von dem Hohlzylinder auf die Membran übertragen werden, die sich dadurch verformt und somit eine Änderung ihres Krümmungsradius erfährt. Indem die Membran ihren Krümmungsradius ändert, ändert die Membran ihre Refraktion, sodass eine Akkommodation des Auges auf Gegenstände in der Ferne oder in der Nähe erreichbar ist. Bei einer Bewegung des Hohlzylinders senkrecht zu der optischen Achse stößt die Rhexis an die Außenfläche an, so dass der Hohlzylinder gegen ein Verrutschen gesichert ist.

Es ist bevorzugt, dass in dem Kopplungszustand die Achse des Hohlzylinders im Wesentlichen parallel zu der optischen Achse ist. Besonders bevorzugt fallen in dem Kopplungszustand die Achse des Hohlzylinders und die optische Achse zusammen.

Es ist bevorzugt, dass die Auflagefläche unmittelbar benachbart zu dem proximalen Ende der Außenfläche angeordnet ist.

Es ist bevorzugt, dass die Außenfläche die Form der Oberfläche eines Zylinders hat. Dabei ist besonders bevorzugt, dass die Achse des Zylinders mit der Achse des Hohlzylinders zusammenfällt.

Die Auflagefläche hat bevorzugt die Form eines Kreisrings. Die Normale des Kreisrings ist besonders bevorzugt parallel zu der Achse des Zylinders.

Der Winkel zwischen der Außenfläche und der Auflagefläche beträgt bevorzugt 80° bis 135°. Besonders bevorzugt beträgt der Winkel zwischen der Außenfläche und der Auflagefläche 80° bis 100° oder 90°.

Es ist bevorzugt, dass der zweite Linsenteil eine Nutfläche aufweist, die der Auflagefläche zugewandt und unmittelbar benachbart zu einem distalen Ende der Außenfläche angeordnet ist, wobei die Außenfläche, die Auflagefläche und die Nutfläche eine Nut begrenzen. Dabei ist besonders bevorzugt, dass die Nut in Umfangsrichtung bezüglich der optischen Achse umlaufend ist und sich insbesondere entlang des gesamten Umfangs des Hohlzylinders erstreckt.

Der zweite Linsenteil weist bevorzugt einen Klemmring auf, der eingerichtet ist, benachbart zu der Außenfläche und der Auflagefläche angeordnet zu werden, und somit eingerichtet ist, den Kapselsack einzuklemmen. Dabei ist denkbar, dass der Kapselsack zwischen der Auflagefläche und dem Klemmring eingeklemmt ist. Indem der Kapselsack eingeklemmt ist, ist der Hohlzylinder besonders fest mit dem Kapselsack gekoppelt. Dadurch kann eine Kraft besonders effektiv von dem Kapselsack auf den Hohlzylinder übertragen werden. Zudem ist der Hohlzylinder besonders fest gegen das Verrutschen gesichert. Zudem ist denkbar, dass der Klemmring die Außenfläche kontaktiert. Es ist besonders bevorzugt, dass der Klemmring in die Nut eingebracht ist.

Der Klemmring ist bevorzugt ein Spaltring. Der Spaltring lässt sich vorteilhaft einfach an der Außenfläche und der Auflagefläche anordnen. Es ist bevorzugt, dass der Spaltring ein erstes Längsende und ein zweites Längsende aufweist, die einen Spalt des Spaltrings begrenzen, wobei der Spaltring im Bereich des ersten Längsendes eine erste Aussparung und im Bereich des zweiten Längsendes eine zweite Aussparung aufweist. In jede der beiden Aussparungen kann beispielsweise jeweils ein Stab eingebracht werden. Indem die beiden Stäbe voneinander entfernt werden, kann der Spaltring gespreizt werden, wodurch der Spaltring einfach an der Außenfläche und der Auflagefläche angeordnet werden kann. Es ist besonders bevorzugt, dass der Spaltring an seinem ersten Längsende eine erste Verdickung, in der die erste Aussparung angeordnet ist, und an seinem zweiten Längsende eine zweite Verdickung aufweist, in der die zweite Aussparung angeordnet ist. Dadurch steht den Aussparungen mehr Platz zur Verfügung als in dem Fall, dass die Verdickungen nicht vorgesehen sind.

Es ist bevorzugt, dass die Außenfläche von einem Gewinde gebildet ist, das sich bis zu dem distalen Ende erstreckt. Es ist hier denkbar, dass eine Schraubenmutter auf das Gewinde geschraubt wird, nachdem der Holzylinder in den Kapselsack eingesetzt wurde, und der Kapselsack zwischen der Auflagefläche und der Schraubenmutter eingeklemmt wird.

Es ist bevorzugt, dass der zweite Linsenteil eine eine Nutfläche aufweisende Schraubenmutter aufweist, die auf das Gewinde geschraubt ist, wodurch die Nutfläche der Außenfläche zugewandt und unmittelbar benachbart zu der Außenfläche angeordnet ist und zusammen mit der Auflagefläche und der Außenfläche die Nut begrenzt. Durch ein Schrauben der Schraubenmutter in Richtung zu dem proximalen Ende des Hohlzylinders hin kann die Nut verschmälert werden und der Kapselsack besonders fest mit dem Hohlzylinder gekoppelt werden. Dadurch ist der Hohlyzlinder auch besonders fest gegen das Verrutschen gesichert. Es ist besonders bevorzugt, dass die Schraubenmutter von einem Ring gebildet ist, der eine Schraubenmutteraussparung aufweist. Ein Stab kann in die Schraubenmutteraussparung eingebracht werden und mittels des Stabs kann die Schraubenmutter gedreht werden.

Es ist bevorzugt, dass der zweite Linsenteil einen Offenzustand und einen Geschlossenzustand hat, in dem der zweite Linsenteil eine Nutfläche aufweist, die der Auflagefläche zugewandt und unmittelbar benachbart zu einem distalen Ende der Außenfläche angeordnet ist sowie zusammen mit der Außenfläche und der Auflagefläche eine Nut begrenzt, wobei in dem Offenzustand die Nutfläche nicht vorhanden ist und die Nut nicht ausgebildet ist. Dadurch lässt sich das distale Ende des Hohlzylinders in dem Offenzustand des zweiten Linsenteils einfach in den herausgeschnittenen Teil des Kapselsacks einbringen. Anschließend lässt sich der Hohlzylinder besonders fest an dem Kapselsack anbringen, indem der zweite Linsenteil in den Geschlossenzustand gebracht wird. Der zweite Linsenteil weist besonders bevorzugt ein Formgedächtnismaterial mit einer Übergangstemperatur auf, wobei der zweite Linsenteil eingerichtet ist, durch ein Überschreiten der Übergangstemperatur von dem Offenzustand in den Geschlossenzustand gebracht zu werden. Beispielsweise kann es sich bei der Übergangstemperatur um eine Glasübergangstemperatur oder eine Schmelztemperatur handeln und der Hohlzylinder kann unterhalb der Übergangstemperatur unter einer mechanischen Vorspannung stehen. Durch das Überschreiten der Übergangstemperatur verliert der zweite Linsenteil die mechanische Vorspannung zumindest teilweise und gelangt somit in den Geschlossenzustand. Die Übergangstemperatur kann so gewählt sein, dass sie oberhalb der Körpertemperatur liegt, also beispielsweise höher als 42°C. Durch Bestrahlen mit elektromagnetischer Strahlung, beispielsweise mittels eines Lasers, kann der zweite Linsenteil auf Temperaturen oberhalb der Übergangstemperatur erwärmt werden.

Es ist bevorzugt, dass der Hohlraum mit einem Gas, einem Öl, insbesondere einem Silikonöl, oder einem Gel, insbesondere einem Silikongel, gefüllt ist. Dies ist vorteilhaft, da Gas einen anderen Refraktionsindex als ein aus einem Acrylpolymerwerkstoff gebildeter Optikkörper oder eine Membran aufweist. Der Refraktionsindex eines Acrylpolymers liegt bei etwa 1,47 bis 1,55, der Refraktionsindex eines Gases wie zum Beispiel Luft liegt bei etwa 1,00003, wobei diese Werte bei der Wellenlänge von 589 nm der Natrium-D-Linie gelten. Somit lässt sich durch Einsatz eines Gases in dem Hohlraum eine Differenz des Refraktionsindex von etwa 0,5 erreichen. Wenn sich durch eine Verlagerung des Hohlzylinders und der Membran relativ zum Optikkörper die Höhe des Hohlraumes verändert, sodass sich das vorhandene Gasvolumen zwischen der Membran und der vorderen Optikkörperoberfläche verändert, hat dies eine Veränderung der Brechkraft der gesamten Intraokularlinse zur Folge. Bereits durch eine geringe Akkommodation und somit Verlagerung von Hohlzylinder und Membran relativ zum Optikkörper und damit Änderung des Krümmungsradius der Membran kann eine relativ große Veränderung der Refraktion erreicht werden. Auch für das Öl und das Gel kann ein anderer Refraktionsindex wie für den Optikkörper und die Membran gewählt werden.

Im Folgenden wird anhand der beigefügten schematischen Zeichnungen die Erfindung näher erläutert.
Figur 1 zeigt einen Schnitt durch eine erste Ausführungsform einer erfindungsgemäßen Intraokularlinse.
Figur 2 zeigt eine perspektivische Ansicht der ersten Ausführungsform.
Figur 3 zeigt einen Schnitt durch eine zweite Ausführungsform der Intraokularlinse.
Figur 4 zeigt eine perspektivische Ansicht eines Bauteils der zweiten Ausführungsform.
Figur 5 zeigt einen Schnitt durch eine dritte Ausführungsform der Intraokularlinse.
Figur 6 zeigt eine andere Perspektive des Schnitts aus Figur 5.
Figur 7 zeigt einen Schnitt einer vierten Ausführungsform der Intraokularlinse in einem Offenzustand.
Figur 8 zeigt einen Schnitt der vierten Ausführungsform in einem Geschlossenzustand.
Figur 9 zeigt einen Schnitt durch einen Hohlzylinder einer fünften Ausführungsform der Intraokularlinse.

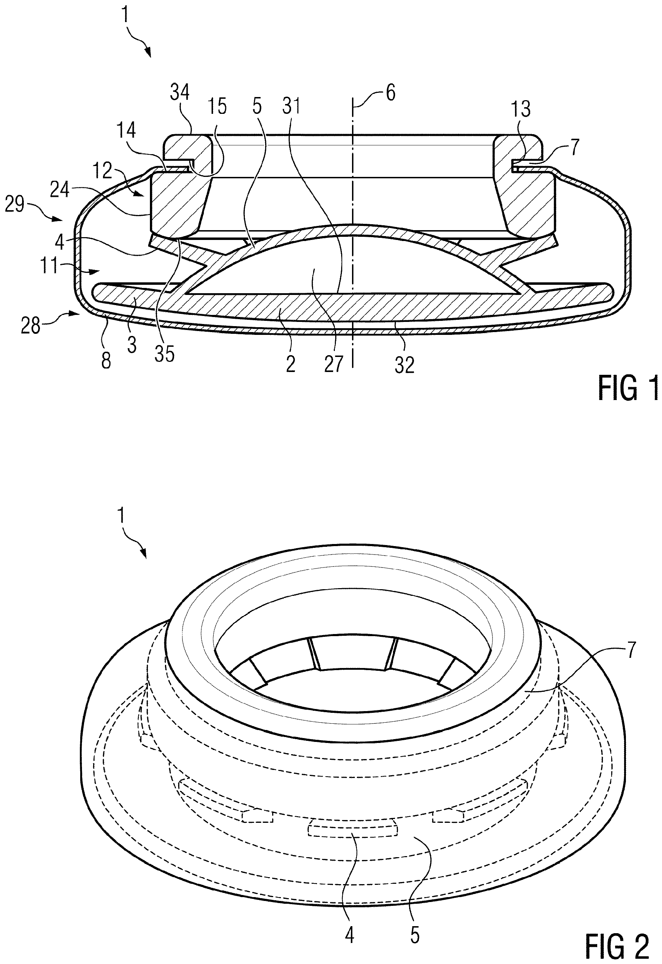

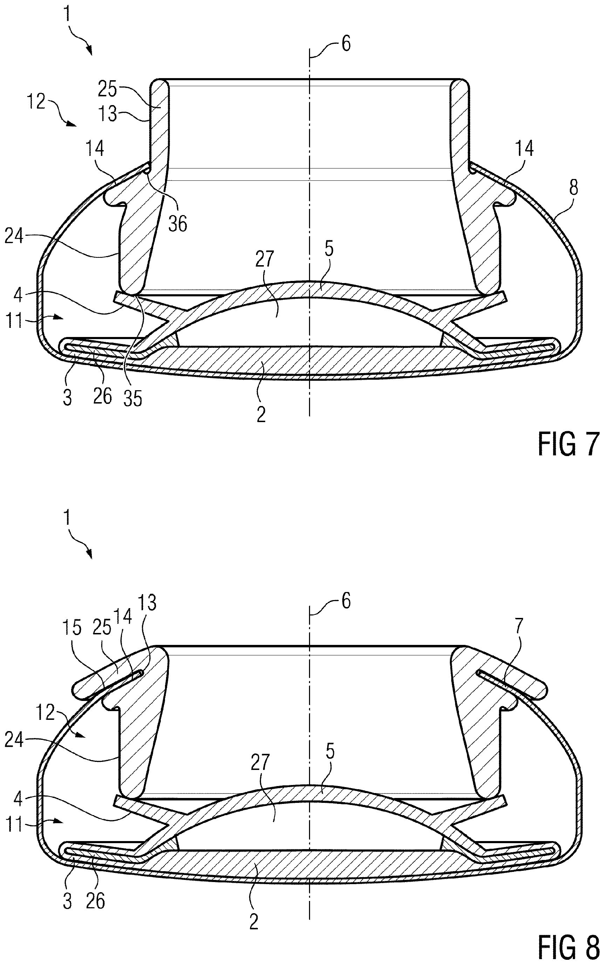

Wie es aus Figuren 1 bis 9 ersichtlich ist, weist eine akkommodative Intraokularlinse 1 zum Einbringen in den Kapselsack 8 eines Auges auf:
- einen ersten Linsenteil 11, welcher aufweist:
   - einen für Licht transparenten Optikkörper 2 mit einer optischen Achse 6, einer distalen Optikkörperoberfläche 31 und einer proximalen Optikkörperoberfläche 32,
   - eine mit dem Optikkörper 2 fest verbundene Haptik 3,
   - eine mit der Haptik 3 und/oder dem Optikkörper 2 fest verbundene flexible Membran 5, die benachbart zur distalen Optikkörperoberfläche 31 angeordnet ist, zusammen mit der distalen Optikkörperoberfläche 31 einen Hohlraum 27 begrenzt und transparent für das Licht ist, und
- einen zweiten Linsenteil 12, der einen Hohlzylinder 24 mit einem distalen Ende 34 und einem proximalen Ende 35 aufweist und der sich mit dem ersten Linsenteil 11 lösbar koppeln lässt, wodurch die Intraokularlinse 1 in einen Kopplungszustand bringbar ist, in dem der zweite Linsenteil 12 an einer distalen Seite 29 des ersten Linsenteils 11 angeordnet ist und der Hohlzylinder 24 eingerichtet ist, durch ein Längsverlagern des Hohlzylinders 24 parallel zu der optischen Achse 6, die Membran 5 zu verformen, wobei der Hohlzylinder 24 an seiner Außenseite eine Außenfläche 13, die ein in Radialrichtung bezüglich der Achse des Hohlzylinders 24 außenliegendes Ende des Hohlzylinders 24 ist, und eine Auflagefläche 14 aufweist, die benachbart zu einem proximalen Ende der Außenfläche 13 angeordnet ist und mit der Außenfläche 13 einen Winkel einschließt, der kleiner als 180° ist. Es ist denkbar, dass in dem Kopplungszustand die Achse des Hohlzylinders 24 im Wesentlichen parallel zu der optischen Achse 6 ist, wie es auch aus Figuren 1 bis 9 ersichtlich ist. Der Hohlraum 27 kann hermetisch abgeschlossen sein. Zudem kann
der Hohlraum 27 mit einem Gas, einem Öl, insbesondere einem Silikonöl, oder einem Gel, insbesondere einem Silikongel, gefüllt sein. Figuren 7 und 8 zeigen, dass der Hohlraum 27 ein Reservoir 26 aufweisen kann, das in der Haptik 3 angeordnet ist. Bei dem Verformen der Membran kann das Gas, das Öl oder das Gel aus dem Reservoir 26 heraus und in das Reservoir 26 hinein strömen.

Die Auflagefläche 14 kann unmittelbar benachbart zu dem proximalen Ende der Außenfläche 13 angeordnet ist. Dies ist beispielhaft in Figuren 1 bis 3, 5, 6, 8 und 9 dargestellt. Beispielsweise kann der Winkel einen Betrag von 80° bis 135° aufweisen, wie es in beispielsweise bei den in den Figuren 1 bis 3 und 5 bis 9 gezeigten Ausführungsformen der Fall ist. Beispielsweise kann der Winkel auch von 80° bis 100° betragen, wie es beispielsweise bei den in den Figuren 1 bis 3, 5, 6, 8 und 9 gezeigten Ausführungsformen der Fall ist. In einem anderen Beispiel kann der Winkel 90° betragen, wie es beispielsweise bei den in den Figuren 1 bis 3, 5, 6 und 9 gezeigten Ausführungsformen der Fall ist.

Es ist denkbar, dass sich die Außenfläche 13 und die Auflagefläche 14 entlang des gesamten Umfangs des Hohlyzlinders 24 erstrecken. Die Außenfläche 13 kann die Form der Oberfläche eines Zylinders haben, wobei die Achse des Zylinders mit der Achse des Hohlzylinders 24 zusammenfallen kann. Bei der Auflagefläche 14 ist denkbar, dass sie die Form eines Kreisrings hat. Die Normale des Kreisrings kann parallel zu der Achse des Zylinders sein.

Figuren 1 bis 3 und 5 bis 8 zeigen, dass der erste Linsenteil 11 eine Mehrzahl von Biegeelementen 4 aufweist, die außerhalb des Hohlraums 27 an der Membran 5 angebracht sind und die in dem Kopplungszustand das proximale Ende 35 des Hohlzylinders 24 kontaktieren. Die Biegeelemente 4 können in Umfangsrichtung bezüglich der optischen Achse 6 an der Membran 5 als radial nach außen gerichtete Biegeelemente 4 in Form eines einseitig an der Membran 5 fixierten Biegebalkens vorgesehen sein. Die Biegeelemente 4 können in gleichem Horizontalwinkel oder im Azimut zueinander angeordnet sein.

Der Hohlzylinder 24 sitzt im Kopplungszustand mit seinem proximalen Ende 35 auf den Biegeelementen 4.

Figuren 1 bis 3 und 5 bis 9 zeigen einen Zustand der Intraokularlinse 1, in dem die Intraokularlinse 1 in dem Kopplungszustand ist und in dem die Intraokularlinse 1 in den Kapselsack 8 eingebracht ist. Aus dem Kapselsack 8 wurde ein im Wesentlichen kreisförmiger Teil herausgeschnitten, so dass eine im Wesentlichen kreisförmige Schnittkante in dem Auge verblieben ist. Das distale Ende 34 des Hohlzylinders 24 ist in dem herausgeschnittenen Teil des Kapselsacks 8 angeordnet.

Figuren 1 bis 3 und 5 bis 8 zeigen, dass der zweite Linsenteil 12 eine Nutfläche 15 aufweisen kann, die der Auflagefläche 14 zugewandt und unmittelbar benachbart zu einem distalen Ende der Außenfläche 13 angeordnet ist, wobei die Außenfläche 13, die Auflagefläche 14 und die Nutfläche 15 eine Nut 7 begrenzen. Dabei ist denkbar, dass die Nut 7 in Umfangsrichtung bezüglich der optischen Achse 6 umlaufend ist. Zudem ist denkbar, dass sich die Nut 7 entlang des gesamten Umfangs des Hohlzylinders 24 erstreckt. Figuren 1 bis 3, 5 und 6 zeigen, dass die Auflagefläche 14 und die Nutfläche 15 parallel zueinander angeordnet sein können. Die Nut 7 kann beispielsweise in Richtung der Achse des Hohlzylinders 24 eine Breite von 200 µm bis 400 pm, insbesondere von 250 µm bis 350 µm, haben.

Gemäß einer ersten Ausführungsform der Intraokularlinse 1, wie sie in Figuren 1 und 2 dargestellt ist, kann der Kapselsack 8 lediglich in die Nut 7 eingebracht werden, ohne dass ein weiteres Bauteil in die Nut 7 eingebracht wurde. In diesem Fall weist der erste Linsenteil 11 neben dem Hohlzylinder 24 kein weiteres Bauteil auf.

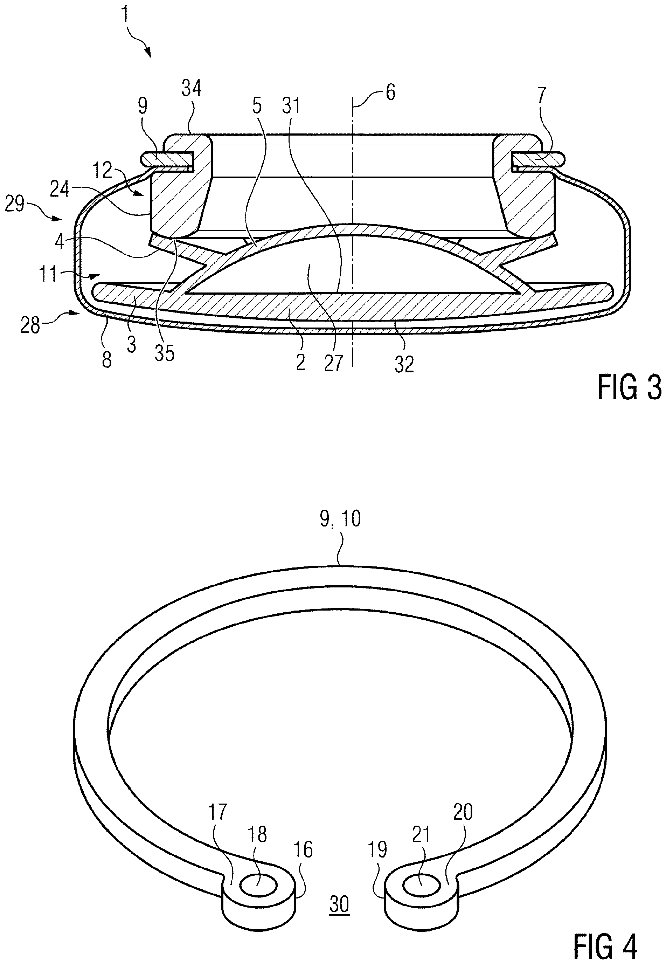

Gemäß einer zweiten Ausführungsform der Intraokularlinse 1 gemäß Figuren 3 und 4 kann der zweite Linsenteil 12 einen Klemmring 9 aufweisen, der eingerichtet ist, in die Nut 7 eingebracht zu werden, und somit eingerichtet ist, den Kapselsack 8 in der Nut 7 einzuklemmen.

Gemäß einer ersten Variante der zweiten Ausführungsform kann der Klemmring 9 ein Spaltring 10 sein, wie er in Figur 4 beispielhaft dargestellt ist. Der Spaltring 9 weist ein erstes Längsende 16 und ein zweites Längsende 19 auf, die einen Spalt 30 des Spaltrings 10 begrenzen. Figur 4 zeigt, dass der Spaltring 10 im Bereich des ersten Längsendes 16 eine erste Aussparung 18 und im Bereich des zweiten Längsendes 19 eine zweite Aussparung 21 aufweisen kann. Die erste Aussparung 18 und die zweite Aussparung 21 können dabei in einer Seite eingebracht sein, die, wenn der Spaltring 10 in der Nut 7 angeordnet ist und die Intraokularlinse 1 in dem Kopplungszustand ist, von dem ersten Linsenteil 11 abgewandt angeordnet ist. Zudem kann die Normale der Seite parallel zu der Achse des Hohlzylinders 24 sein. Die erste Aussparung 18 und die zweite Aussparung 21 können jeweils als ein Durchgangsloch ausgebildet sein. Zudem ist in Figur 4 erkennbar, dass der Spaltring 10 an seinem ersten Längsende 16 eine erste Verdickung 17, in der die erste Aussparung 18 angeordnet ist, und an seinem zweiten Längsende 19 eine zweite Verdickung 20 aufweisen kann, in der die zweite Aussparung 21 angeordnet ist.

Gemäß einer zweiten Variante der zweiten Ausführungsform kann der Klemmring 9 ohne einen Spalt ausgebildet sein, d.h. der Klemmring 9 erstreckt sich über einen Winkel von 360°.

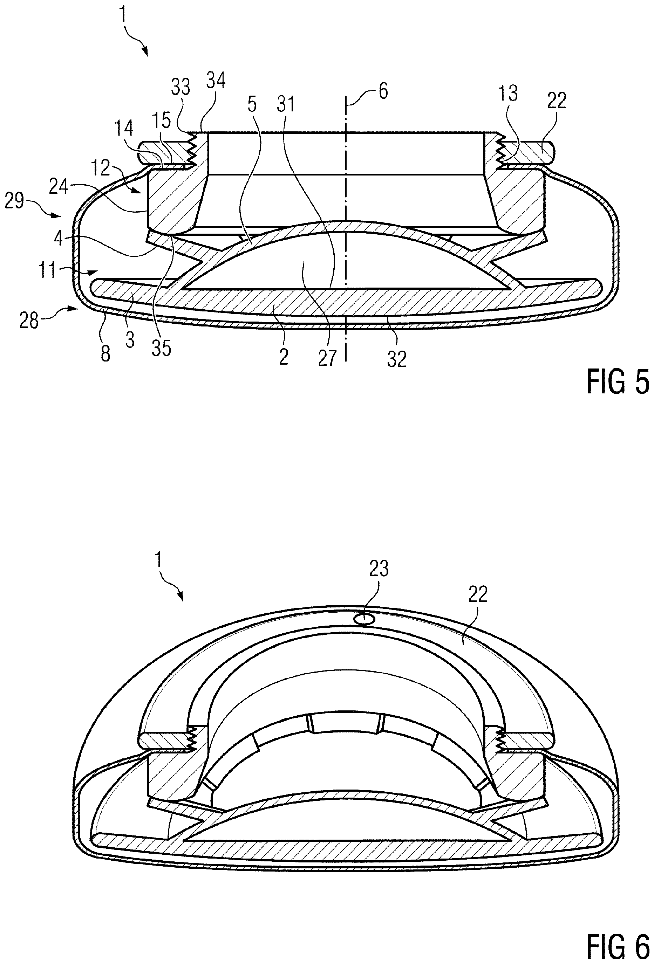

Figuren 5 und 6 zeigen eine dritte Ausführungsform der Intraokularlinse 1. Hier ist die Außenfläche 13 von einem Gewinde 33 gebildet, dass sich bis zu dem distalen Ende 34 erstreckt. Der zweite Linsenteil 12 kann eine Schraubenmutter 22 aufweisen, die auf das Gewinde 33 geschraubt ist, wodurch die Nut 7 in Richtung der optischen Achse 6 und an ihrem distalen Ende von der Schraubenmutter 22 begrenzt ist. Die Unterseite der Schraubenmutter 22 bildet somit die Nutfläche 15. Indem die Schraubenmutter 22 in Richtung zu der Auflagefläche 14 geschraubt wird, verkürzt sich die Erstreckung der Nut in Richtung der Achse des Hohlzylinders 24 und ein Teil des Kapselsackes 8 kann von der Auflagenfläche 14 und der Nutfläche 15 formschlüssig und/oder kraftschlüssig in seiner Lage fixiert werden. Figur 6 zeigt, dass die Schraubenmutter 22 von einem Ring gebildet sein kann, der eine Schraubenmutteraussparung 23 aufweist. Die Schraubenmutteraussparung 23 kann dabei in einer Seite eingebracht sein, die, wenn die Schraubenmutter 22 in der Nut 7 angeordnet ist und die Intraokularlinse 1 in dem Kopplungszustand ist, von dem ersten Linsenteil 11 abgewandt angeordnet ist. Zudem kann die Normale der Seite parallel zu der Achse des Hohlzylinders 24 sein. Die Schraubenmutteraussparung 23 kann als ein Durchgangsloch ausgebildet sein.

Figuren 7 und 8 zeigen eine vierte Ausführungsform für die Intraokularlinse 1, bei der der zweite Linsenteil 12 einen Offenzustand und einen Geschlossenzustand hat, in dem der zweite Linsenteil 12 eine Nutfläche 15 aufweist, die der Auflagefläche 14 zugewandt und unmittelbar benachbart zu einem distalen Ende der Außenfläche 13 angeordnet ist sowie zusammen mit der Außenfläche 13 und der Auflagefläche 14 eine Nut 7 begrenzt, wobei in dem Offenzustand die Nutfläche 15 nicht vorhanden ist und die Nut 7 nicht ausgebildet ist. Dazu kann der Hohlzylinder 24 einen Kragen 25 aufweisen, der von dem verbliebenen Hohlzylinder 24 absteht und auf seiner Außenseite die Nutfläche 15 bildet. Der Kragen 25 kann sich, gesehen von der Achse des Hohlzylinders 24, entlang eines Winkels von 360° erstrecken. Die Auflagefläche 14 kann in einer Richtung von radial innen nach radial außen in Richtung zu einem proximalen Ende 35 des Hohlzylinders 24 geneigt sein.

Es ist denkbar, dass, wie in Figur 7 dargestellt, bei der vierten Ausführungsform der Hohlzylinder 24 in dem Offenzustand eine Rille 36 aufweist, die die Außenfläche 13 und die Auflagefläche 14 voneinander trennt, so dass die Außenfläche 13 und die Auflagefläche 14 nicht unmittelbar zueinander benachbart sind. In dem Geschlossenzustand ist es denkbar, dass die Rille 36 zu der Außenfläche 13 wird und dass die Außenfläche 13 zu der Nutfläche 15 wird, wie es in Figur 8 dargestellt ist.

Bei der vierten Ausführungsform ist es denkbar, dass der zweite Linsenteil 12 ein Formgedächtnismaterial mit einer Übergangstemperatur aufweist, wobei der zweite Linsenteil 12 eingerichtet ist, durch ein Überschreiten der Übergangstemperatur von dem Offenzustand in den Geschlossenzustand gebracht zu werden. Die Übergangstemperatur kann beispielsweise höher als 42°C sein. Das Formgedächtnismaterial kann beispielsweise durch Bestrahlen mit Laserstrahlung auf eine Temperatur oberhalb der Übergangstemperatur erwärmt werden. Zudem ist denkbar, dass bei der Übergangstemperatur ein Glasübergang oder ein Schmelzen des Formgedächtnismaterials stattfindet. Unterhalb der Übergangstemperatur kann das Formgedächtnismaterial unter einer mechanischen Vorspannung stehen, die zumindest teilweise bei dem Überschreiten der Übergangstemperatur verloren geht.

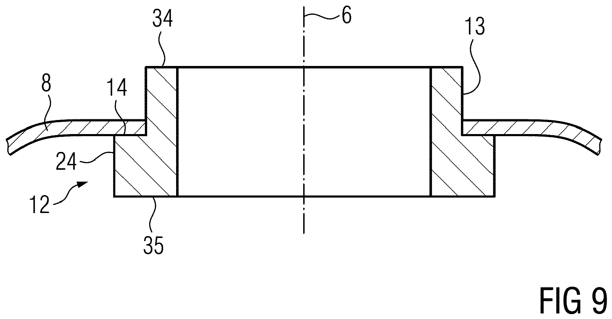

Alternativ dazu, die Nut 7 vorzusehen, ist es denkbar, dass in Radialrichtung bezüglich der Achse des Hohlzylinders außen an dem Hohlzylinder 24 und an dem distalen Ende 34 des Hohlzylinders 24 lediglich die Außenfläche 13 und die Auflagefläche 14 vorsehen sind. Dies ist beispielsweise in Figur 9 für eine fünfte Ausführungsform der Intraokularlinse 1 dargestellt. Bei der fünften Ausführungsform ist es denkbar, dass der zweite Linsenteil 12 den Klemmring 9 aufweist.

### Bezugszeichenliste

1 Intraokularlinse
2 Optikkörper
3 Haptik
4 Biegeelement
5 Membran
6 optische Achse
7 Nut
8 Kapselsack
9 Klemmring
10 Spaltring
11 erster Linsenteil
12 zweiter Linsenteil
13 Außenfläche
14 Auflagefläche
15 Nutfläche
16 erstes Längsende
17 erste Verdickung
18 erste Aussparung
19 zweites Längsende
20 zweite Verdickung
21 zweite Aussparung
22 Schraubenmutter
23 Schraubenmutteraussparung
24 Hohlzylinder
25 Kragen
26 Reservoir
27 Hohlraum
28 proximale Seite des ersten Linsenteils
29 distale Seite des ersten Linsenteils
30 Spalt
31 distale Optikkörperoberfläche
32 proximale Optikkörperoberfläche
33 Gewinde
34 distale Ende des Hohlzylinders
35 proximale Ende des Hohlzylinders
36 Rille

## Patentansprüche

1. Akkommodative Intraokularlinse zum Einbringen in den Kapselsack (8) eines Auges, wobei die Introkularlinse (1) aufweist:
- einen ersten Linsenteil (11), welcher aufweist:
- einen für Licht transparenten Optikkörper (2) mit einer optischen Achse (6), einer distalen Optikkörperoberfläche (31) und einer proximalen Optikkörperoberfläche (32),
- eine mit dem Optikkörper (2) fest verbundene Haptik (3),
- eine mit der Haptik (3) und/oder dem Optikkörper (2) fest verbundene flexible Membran (5), die benachbart zur distalen Optikkörperoberfläche (31) angeordnet ist, zusammen mit der distalen Optikkörperoberfläche (31) einen Hohlraum (27) begrenzt und transparent für das Licht ist, und
- einen zweiten Linsenteil (12), der einen Hohlzylinder (24) mit einem distalen Ende (34) und einem proximalen Ende (35) aufweist und der sich mit dem ersten Linsenteil (11) lösbar koppeln lässt, wodurch die Intraokularlinse (1) in einen Kopplungszustand bringbar ist, in dem der zweite Linsenteil (12) an einer distalen Seite (29) des ersten Linsenteils (11) angeordnet ist und der Hohlzylinder (24) eingerichtet ist, durch ein Längsverlagern des Hohlzylinders (24) parallel zu der optischen Achse (6), die Membran (5) zu verformen, wobei der erste Linsenteil (11) eine Mehrzahl von Biegeelementen (4) aufweist, die außerhalb des Hohlraums (27) an der Membran (5) angebracht sind und die in dem Kopplungszustand ein proximales Ende des Hohlzylinders (24) kontaktieren und der Hohlzylinder (24) an seiner Außenseite eine Außenfläche (13) aufweist, die ein in Radialrichtung bezüglich der Achse des Hohlzylinders (24) außenliegendes Ende des Hohlzylinders (24) ist, wobei der Hohlzylinder (24) eine Auflagefläche (14) aufweist, die eingerichtet ist, von dem Kapselsack (8) kontaktiert zu werden, benachbart zu einem proximalen Ende der Außenfläche (13) angeordnet ist und mit der Außenfläche (13) einen Winkel einschließt, der kleiner als 180° ist.

2. Intraokularlinse gemäß Anspruch 1, wobei in dem Kopplungszustand die Achse des Hohlzylinders (24) im Wesentlichen parallel zu der optischen Achse (6) ist.

3. Intraokularlinse gemäß Anspruch 1 oder 2, wobei die Auflagefläche (14) unmittelbar benachbart zu dem proximalen Ende der Außenfläche (13) angeordnet ist.

4. Intraokularlinse gemäß einem der Ansprüche 1 bis 3, wobei der zweite Linsenteil (12) eine Nutfläche (15) aufweist, die der Auflagefläche (14) zugewandt und unmittelbar benachbart zu einem distalen Ende der Außenfläche (13) angeordnet ist, wobei die Außenfläche (13), die Auflagefläche (14) und die Nutfläche (15) eine Nut (7) begrenzen.

5. Intraokularlinse gemäß einem der Ansprüche 1 bis 4, wobei der zweite Linsenteil (12) einen Klemmring (9) aufweist, der eingerichtet ist, benachbart zu der Außenfläche (13) und der Auflagefläche (14) angeordnet zu werden, und somit eingerichtet ist, den Kapselsack (8) einzuklemmen.

6. Intraokularlinse gemäß Anspruch 5, wobei der Klemmring (9) ein Spaltring (10) ist.

7. Intraokularlinse gemäß einem der Ansprüche 1 bis 4, wobei die Außenfläche (13) von einem Gewinde (33) gebildet ist, das sich bis zu dem distalen Ende (34) erstreckt.

8. Intraokularlinse gemäß einem der Ansprüche 1 bis 3, wobei der zweite Linsenteil (12) einen Offenzustand und einen Geschlossenzustand hat, in dem der zweite Linsenteil (12) eine Nutfläche (15) aufweist, die der Auflagefläche (14) zugewandt und unmittelbar benachbart zu einem distalen Ende der Außenfläche (13) angeordnet ist sowie zusammen mit der Außenfläche (13) und der Auflagefläche (14) eine Nut (7) begrenzt, wobei in dem Offenzustand die Nutfläche (15) nicht vorhanden ist und die Nut (7) nicht ausgebildet ist, wobei der zweite Linsenteil (12) ein Formgedächtnismaterial mit einer Übergangstemperatur aufweist, wobei der zweite Linsenteil (12) eingerichtet ist, durch ein Überschreiten der Übergangstemperatur von dem Offenzustand in den Geschlossenzustand gebracht zu werden.

## Claims

1. Accommodative intraocular lens for insertion into the capsular bag (8) of an eye, the intraocular lens (1) having:
- a first lens part (11), which has:
- an optic (2), which is transparent to light and has an optical axis (6), a distal optic surface (31) and a proximal optic surface (32),
- a haptic (3) firmly connected to the optic (2),
- a flexible membrane (5), which is firmly connected to the haptic (3) and/or the optic (2), is arranged adjacent to the distal optic surface (31), delimits a cavity (27) together with the distal optic surface (31) and is transparent to the light, and
- a second lens part (12), which has a hollow cylinder (24) with a distal end (34) and a proximal end (35) and which can be detachably coupled to the first lens part (11), whereby the intraocular lens (1) is able to be brought into a coupling state in which the second lens part (12) is arranged on a distal side (29) of the first lens part (11) and the hollow cylinder (24) is configured to deform the membrane (5) by way of a longitudinal displacement of the hollow cylinder (24) parallel to the optical axis (6), wherein the first lens part (11) has a plurality of bending elements (4), which are attached to the membrane (5) outside the cavity (27) and which contact a proximal end of the hollow cylinder (24) in the coupling state, and the hollow cylinder (24) has on its outer side an outer surface (13), which is an outer end of the hollow cylinder (24) in the radial direction with respect to the axis of the hollow cylinder (24), wherein the hollow cylinder (24) has a bearing surface (14), which is configured to be contacted by the capsular bag (8), is arranged adjacent to a proximal end of the outer surface (13) and includes an angle of less than 180° with the outer surface (13).

2. Intraocular lens according to Claim 1, wherein the axis of the hollow cylinder (24) is substantially parallel to the optical axis (6) in the coupling state.

3. Intraocular lens according to Claim 1 or 2, wherein the bearing surface (14) is arranged directly adjacent to the proximal end of the outer surface (13).

4. Intraocular lens according to one of Claims 1 to 3, wherein the second lens part (12) has a groove surface (15), which faces the bearing surface (14) and is arranged directly adjacent to a distal end of the outer surface (13), with the outer surface (13), the bearing surface (14) and the groove surface (15) delimiting a groove (7).

5. Intraocular lens according to one of Claims 1 to 4, wherein the second lens part (12) has a clamping ring (9), which is configured to be arranged adjacent to the outer surface (13) and the bearing surface (14) and is thus configured to clamp the capsular bag (8).

6. Intraocular lens according to Claim 5, wherein the clamping ring (9) is a split ring (10).

7. Intraocular lens according to one of Claims 1 to 4, wherein the outer surface (13) is formed by a thread (33), which extends up to the distal end (34).

8. Intraocular lens according to one of Claims 1 to 3, wherein the second lens part (12) has an open state and a closed state, in which the second lens part (12) has a groove surface (15), which faces the bearing surface (14) and is arranged directly adjacent to a distal end of the outer surface (13) and which delimits a groove (7) together with the outer surface (13) and the bearing surface (14), with the groove surface (15) not being present and the groove (7) not being formed in the open state, wherein the second lens part (12) comprises a shape memory material with a transition temperature, with the second lens part (12) being configured to be brought from the open state to the closed state when the transition temperature is exceeded.

## Revendications

1. Lentille intraoculaire accommodative destinée à être insérée dans le sac capsulaire (8) d'un œil, la lentille intraoculaire (1) comportant :
- une première partie de lentille (11) qui comporte :
- un corps optique (2) qui est transparent à la lumière et qui possède un axe optique (6), une surface de corps optique distale (31) et une surface de corps optique proximale (32),
- un système haptique (3) relié de manière fixe au corps optique (2),
- une membrane flexible (5) qui est reliée de manière fixe au système haptique (3) et/ou au corps optique (2) et qui est disposée à proximité de la surface de corps optique distale (31), qui délimite conjointement avec la surface de corps optique distale (31) une cavité (27) et qui est transparente à la lumière, et
- une deuxième partie de lentille (12), qui comporte un cylindre creux (24) pourvu d'une extrémité distale (34) et d'une extrémité proximale (35) et qui peut être couplée de manière amovible à la première partie de lentille (11), de sorte que la lentille intraoculaire (1) peut être amenée dans un état de couplage dans lequel la deuxième partie de lentille (12) est disposée sur un côté distal (29) de la première partie de lentille (11) et que le cylindre creux (24) est conçu pour déformer la membrane (5) par déplacement longitudinal du cylindre creux (24) parallèlement à l'axe optique (6), la première partie de lentille (11) comportant une pluralité d'éléments de courbure (4) qui sont fixés à la membrane (5) à l'extérieur de la cavité (27) et qui, dans l'état de couplage, entrent en contact avec une extrémité proximale du cylindre creux (24) et le cylindre creux (24) comportant sur son côté extérieur une surface extérieure (13) qui est une extrémité extérieure du cylindre creux (24) dans la direction radiale par rapport à l'axe du cylindre creux (24), le cylindre creux (24) comportant une surface d'appui (14) qui est conçue pour venir en contact avec le sac capsulaire (8), qui est disposée de manière adjacente à une extrémité proximale de la surface extérieure (13) et qui forme avec la surface extérieure (13) un angle qui est inférieur à 180°.

2. Lentille intraoculaire selon la revendication 1, l'axe du cylindre creux (24) étant, dans l'état de couplage, sensiblement parallèle à l'axe optique (6).

3. Lentille intraoculaire selon la revendication 1 ou 2, la surface d'appui (14) étant disposée de manière directement adjacente à l'extrémité proximale de la surface extérieure (13).

4. Lentille intraoculaire selon l'une des revendications 1 à 3, la deuxième partie de lentille (12) comportant une surface de rainure (15) qui est dirigée vers la surface d'appui (14) et qui est disposée de manière directement adjacente à une extrémité distale de la surface extérieure (13), la surface extérieure (13), la surface d'appui (14) et la surface de rainure (15) délimitant une rainure (7).

5. Lentille intraoculaire selon l'une des revendications 1 à 4, la deuxième partie de lentille (12) comportant un anneau de serrage (9) qui est conçu pour être disposé de manière adjacente à la surface extérieure (13) et à la surface d'appui (14) et qui est ainsi conçu pour serrer le sac capsulaire (8).

6. Lentille intraoculaire selon la revendication 5, la bague de serrage (9) étant une bague fendue (10).

7. Lentille intraoculaire selon l'une des revendications 1 à 4, la surface extérieure (13) étant formée par un filetage (33) qui s'étend jusqu'à l'extrémité distale (34).

8. Lentille intraoculaire selon l'une des revendications 1 à 3, la deuxième partie de lentille (12) présentant un état ouvert et un état fermé dans lequel la deuxième partie de lentille (12) comporte une surface de rainure (15) qui est dirigée vers la surface d'appui (14) et qui est disposée de manière directement adjacente à une extrémité distale de la surface extérieure (13) et qui délimite une rainure (7) conjointement avec la surface extérieure (13) et la surface d'appui (14), la surface de rainure (15) n'étant pas présente, et la rainure (7) n'étant pas formée, à l'état ouvert, la deuxième partie de lentille (12) comportant un matériau à mémoire de forme ayant une température de transition, la deuxième partie de lentille (12) étant conçue pour être amenée de l'état ouvert à l'état fermé par franchissement vers le haut de la température de transition.
